# EUROPÄISCHE PATENTSCHRIFT

(11) **EP 2 643 054 B1**
(45) Veröffentlichungstag und Bekanntmachung des Hinweises auf die Patenterteilung: **18.03.2020**
(21) Anmeldenummer: 11799485.5
(22) Anmeldetag: 23.11.2011
(51) Int. Cl.: A61N 5/10

(54) **ANPASSEN EINER DOSISVERTEILUNGS-EINSTELLUNG FÜR EIN TECHNISCHES GERÄT DER TUMORTHERAPIE**
CUSTOMIZATION OF A DOSE DISTRIBUTION SETTING FOR A TECHNICAL APPLIANCE FOR TUMOUR THERAPY
ADAPTATION D'UN AJUSTEMENT DE DISTRIBUTION DE DOSES POUR UN APPAREIL TECHNIQUE DE TRAITEMENT DES TUMEURS

(30) Priorität: 26.11.2010 DE 102010060847; 27.11.2010 DE 102010062079
(43) Veröffentlichungstag der Anmeldung: 02.10.2013
(73) Patentinhaber: Fraunhofer-Gesellschaft zur Förderung der angewandten Forschung e.V., 80686 München (DE)
(72) Erfinder: KUEFER, Karl-Heinz, 67685 Weilerbach (DE); SCHERRER, Alexander, 67663 Kaiserslautern (DE); MONZ, Michael, 55128 Mainz (DE); SUESS, Philipp, 67663 Kaiserslautern (DE); BORTZ, Michael, 67655 Kaiserslautern (DE)
(74) Vertreter: Leonhard, Frank Reimund
(86) Internationale Anmeldenummer: PCT/IB2011/055249
(87) Internationale Veröffentlichungsnummer: WO 2012/069999

(56) Entgegenhaltungen:
- EP-A1- 2 260 902
- US-A- 6 111 575
- US-A1- 2003 212 325
- US-A1- 2005 111 621
- THIEKE ET AL: "A new concept for interactive radiotherapy planning with multicriteria optimization: First clinical evaluation", RADIOTHERAPY AND ONCOLOGY, ELSEVIER, IRELAND, Bd. 85, Nr. 2, 1. November 2007 (2007-11-01), Seiten 292-298, XP022363696, ISSN: 0167-8140, DOI: 10.1016/J.RADONC.2007.06.020

## Beschreibung

Die Erfindung befasst sich mit einem Verfahren zum Optimieren eines eingestellten Zustands eines Geräts einer Tumorbehandlung. Insbesondere geht es dabei um eine erhebliche Verbesserung eines schon patentierten Systems, vgl. US 7,391,026 B2**.** Die dort offenbarte Steuerung einer Planungsfindung zur Festlegung eines bestmöglichen Plans zur Behandlung eines Patienten, der unter einer Tumorerkrankung leidet, war ein Quantensprung. Es war eine Umkehr der bisher vorgeschlagenen Vorgehensweisen und ist im Allgemeinen unter IMRT im Fachgebiet bekannt geworden. Die "inverse Therapieplanung" genannte Vorgehensweise wurde von Bortfeld vorgeschlagen, vgl. **US '026 (wie oben),** Spalte 1, Zeile 43 ff. Im Laufe der Zeit, unter Anwendung sowie praktischer Erprobung haben sich Verbesserungsmöglichkeiten eröffnet, die Gegenstand dieser Erfindung sind.

Geht man vom genannten Stand der Technik aus, findet der Planer anhand eines Entwurfswerkzeuges einen geeigneten Plan. Der geeignete Plan ist weitreichend und er enthält Einstellparameter zur Einstellung eines (oder: am) therapeutischen Geräts, welches die Tumortherapie später an einem Patienten ausführt. Entsprechend deutlich ist, dass die Einstellung dieses Geräts und die Erstellung oder Ermittlung der Einstellung des Geräts noch keine therapeutische Behandlung und auch keine medizinische Behandlung als solches ist, sondern eine weit davor liegende Vorstufe. Gefunden werden Einstellparameter technischer Natur, mit denen eine Therapie später und an völlig anderer Stelle durchgeführt werden kann. Diese nennt die genannte IMRT einen Plan. Der Plan ist gleichzeitig auch "eine Lösung", ausgewählt aus einer Vielzahl von vorberechneten Plänen oder Lösungen, die alle geeignet sind, aber nur eine davon ist die für den Planer Bestmögliche. Diese eine Bestmögliche aus der Vielzahl schon zur Verfügungen stehenden Plänen auszuwählen, wird Kraft des Entwurfswerkzeuges gemäß US-Patent 7,391,026 möglich.

Gleichwohl gibt es beim Planer noch Verbesserungsbedarf. Dieser Verbesserungsbedarf kann kritische Stellen im Plan betreffen, die im Plan selbst nur so geändert werden können, dass ein anderer Plan ausgewählt wird. Dieser andere Plan wird mit Blick auf eine Verbesserung an einem "Area of Interest" verändert, ist indes wieder einer der vorberechneten Pläne. Auch dieser war bereits berechnet und in der Datenbank zur Verfügung gestellt.

Die Erfindung geht **von der Aufgabe aus,** einem Planer die Möglichkeit zu geben, einen ihm vorliegenden IMRT-Behandlungsplan lokal zu verbessern. Eine lokale Verbesserung umfasst dabei mehrere Begriffsbestimmungen, beispielsweise eine lokale Überdosierung in einem Risikobereich oder eine lokale Unterdosierung in einem Zielbereich. In spezieller Ausbildung kann sich der Zielbereich konkret auf den Tumor beziehen, der mit einer möglichst hohen Dosis zu versehen ist, und in anderer besonderer Ausgestaltung kann sich der Risikobereich auf ein bestimmtes Risikoorgan (räumlich betrachtet) beziehen. Es können aber auch Bereiche im Gewebe davon betroffen sein, die nicht organmäßig abgegrenzt, sondern nur bereichsmäßig umschrieben werden. Möchte man lokale Unzulänglichkeiten verbessern, kann man in der Bedeutungssprache auch von der Beseitigung von "Critical Spots" oder von Critical Spot Remover als Aufgabe ausgehen, die mit der im Folgenden beanspruchten und umschriebenen Erfindung ermöglicht werden. Abstrakter gesprochen besteht die Aufgabe also darin, Critical Spots lokal zu beseitigen (in beiden Richtungen zu Unterdosierungen oder Überdosierungen) und dabei den schon vorliegenden Plan (die schon ausgewählte Lösung) so gering als möglich zu verändern. Es soll gerade keine der vorberechneten anderen Lösungen verwendet werden, sondern die Ausgangslösung soll lokal begrenzt verändert werden.

Die Druckschrift US2005/0111621-A1 offenbart eine Planungsfindung für die Strahlentherapie, wobei der Benutzer Änderungen im DVH-Diagramm erzeugen kann. Mit einer Bedienhilfe können diese Änderungen stufenweise rückgängig gemacht werden.

Es ist die Aufgabe der vorliegenden Anmeldung die Planungsfindung zu verbessern.

Gelöst wird die Aufgabe mit Anspruch 1, der hier als Verfahren zur Anpassung einer Dosisverteilungs-Einstellung einbezogen ist.

Mit der Lösung wird der ursprüngliche Plan zwar lokal verändert, aber nicht der ganze Plan. Der Fachmann würde dabei davon sprechen, dass unter praktischer Beibehaltung des Ausgangsplans eine lokale Stelle, die als klein benannt werden kann, verändert wird. Würde er einen neuen Plan verwenden, würde er die lokalisierten kritischen Bereiche (die Critical Spots) zwar verändern, aber auch verlagern und im Allgemeinen nicht wirklich entfernen. Eine zu globale Veränderung des Plans wird also vermieden. Anders gefasst, die Änderung am "ersten Plan" soll außerhalb des "critical spots" so wenig als möglich verändert werden.

Diese Umschreibung der Lösung orientiert sich an dem Begriff der lokalen Veränderung, respektive der Spot-Veränderung, welcher Spot relativ zum Gesamtvolumen klein ist.

Nachdem im Dosisverteilungs-Plan mit Voxeln gerechnet wird, kann ein lokales/kleines Volumen weniger als 500 Voxel haben. Dieses räumlich begrenzte Volumen kann Spot oder Box genannt werden. Dabei hat ein Voxel typischerweise eine Kantenlänge von 3 mm. Ein bevorzugtes Ausmaß dieses lokalen Spots (Box oder Spot) ist kleiner als 7 x 7 x 7 Voxel in den drei Raumrichtungen, also kleiner als etwa 350 Voxel, oder unter einer Obergrenze von 500 Voxel (Anspruch 5). Anders dargestellt sind weniger als 5% des Volumens betroffen, das als Planvolumen von der Dosisverteilung betroffen ist (Anspruch 6).

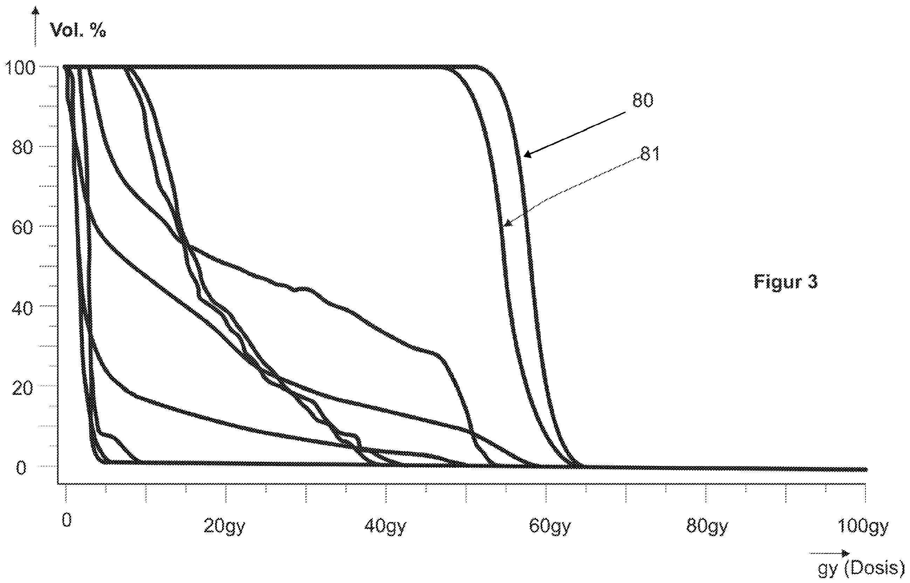

Die Einstellung, welche an dem technischen Gerät oder welche das technische Gerät der Tumortherapie für eine solche Therapie einstellt, ist mit mehreren technischen Parametern verschiedenster Natur einstellbar. Für das IMRT-Verfahren können Multileaf-Köpfe eine erste Einstellung geben, die mit mehreren streifenförmigen Einstellschiebern arbeiten, vgl. Figur 3 und die zugehörige Beschreibung der **US '026,** wie eingangs angegeben. Es können weiter Strahlungsdosen vorgegeben werden, es können Rotationswinkel für einen rotierenden Kopf vorgegeben werden, Verweildauern des rotierenden Kopfes an bestimmten Stellen und natürlich auch Kombinationen der Multileaf-Kopfeinstellung mit den genannten anderen Parametern. In einer groben Übersicht können Zeitdauer (Bestrahlungsdauer), Profil einer jeweiligen Bestrahlung durch den Multileaf-Kopf und unterschiedliche Positionen von Einstrahlwinkeln vorgegeben werden, die unter den Begriff der Einstellung des technischen Geräts zur Tumortherapie fallen. Als Strahlung können Photonen, Elektronen, schwere Ionen oder Protonen verwendet werden, je nach Behandler, Patient und verfügbarem technischen Gerät, wie auch Art und Natur des zu behandelnden (zu bestrahlenden) Tumorgebiets, vgl. erneut **US '026**, dort Absatz [068].

Für diese Behandlung liegt ein Plan vor, zu dem zuvor ausführlich Erläuterungen niedergelegt waren. Dieser erste Plan wird aus einem Datenspeicher, beispielsweise einer geordneten Datenbank ausgelesen und auf einem Bildschirm dargestellt. Bei der Darstellung gibt es eine Vielzahl von Möglichkeiten, die abhängig von der Natur des Nutzers oder seinen bevorzugten Auswahlkriterien sind. In den im Folgenden beschriebenen Beispielen werden ein DVH-Diagramm und drei Schnittdarstellungen (transversal, sagital und frontal) erläutert, die eine gute Übersicht über die Strahlendosen in der räumlichen Verteilung geben und auch eine Art Mittelwert dem Benutzer vermitteln, die eine Gesamteinschätzung eines jeweiligen Plans erlaubt, im Beispiel das DVH-Diagramm. Dargestellt wird im Allgemeinen das gesamte Planvolumen, welches von der Dosisverteilung betroffen ist und in diesem Planvolumen befinden sich sowohl das Zielvolumen (das Tumorgebiet, eines oder mehrere) und auch eine Anzahl von Risiken oder Risikoorganen, die auch als Gebiete vorliegen können, unabhängig von einem physisch abgegrenzten Risikoorgan. Zur Lösung der gestellten Aufgabe wird an einem dargestellten Plan ein Punkt spezifiziert, der einen Dosiswert enthält, der eine unerwünschte Höhe oder eine unerwünschte Schwäche hat. Dieses "Initialvoxel" ist ein Voxel, welches im räumlichen Gebiet der beispielsweise drei senkrecht zueinander liegenden Schnittdarstellungen gelegen ist. Es kann in der sagitalen Darstellung, in der transversalen Darstellung oder in der frontalen Darstellung ausgewählt werden, betrifft aber ein räumliches Gebiet, das um dieses Initialvoxel herum als Box danach definiert wird. Dies geschieht zum Zwecke der Änderung des dargestellten Plans in zumindest einer volumenmäßig spezifizierten Feinheit, die klein gegenüber der Größe des Planvolumens ist. An dieser spezifizierten Stelle und mit der von dem Initialvoxel definierten kleinen/lokalen Gruppe von Voxeln soll eine Änderung erfolgen, entweder aufwärts zu höheren Strahlungsdosen oder herab zu geringeren Strahlungsdosen in dem spezifizierten kleinen Gebiet.

Daraus soll ein kausaler Folgeplan entstehen, der möglichst nahe an dem dargestellten Plan liegt, also das DVH-Diagramm nur wenig ändert, aber die spezifische kleine Stelle als volumenmäßig spezifizierte Feinheit verändern soll.

Das geschieht erfindungsgemäß gerade nicht dadurch, dass ein neuer Plan aus den vorgespeicherten Plänen/Lösungen genommen wird, der in dieser volumenmäßig spezifizierten Feinheit einen dem Wunschwert entsprechende Dosis aufweist, weil dieser Plan mit an Sicherheit grenzender Wahrscheinlichkeit an vielen anderen Stellen sehr große Unterschiede mit sich bringt, die auch ein deutlich anderes DVH bewirken würden, was erfindungsgemäß gerade vermieden werden soll. Durch die Auswahl der bestimmten Stelle mit dem Initialvoxel, das per Cursor identifiziert werden kann und in einer Schicht des Planvolumens liegt, wird die Gruppe von Voxeln festgelegt, die um dieses Initialvoxel herum liegt.

Die Gruppe ist erfindungsgemäß sehr klein (Ansprüche 5, 6) und kann auf Wunsch des Benutzers in seiner Größe definiert werden. Die Auswahl des Initialvoxels in einer Schicht betrifft, wie oben angesprochen, die sagitalen Schichten, die transversalen Schichten oder die frontalen Schichten. Erstrecken wird sich die Gruppe von Voxeln aber nach Festlegung des Initialvoxels im dreidimensionalen Raum, also in allen Schichten, nur identifiziert zu werden braucht es nur in einer Schicht.

Hierbei hat der Nutzer die Wahl, die Position des Initialvoxels in einer der dargestellten Schichten zu bestimmen.

Nach Festlegen des Initialvoxels und der lokalen Gruppe von Voxeln als die spezifizierte Feinheit im wesentlichen größeren Planvolumen wird eine Umrechnung angestoßen. Die Umrechnung geht von dem ersten Plan aus, der dargestellt ist. Er wird in einen ersten Navigationsplan umgerechnet, wobei der erste Plan der Ausgangspunkt (Eingangsplan) ist und die Dosisänderung in der spezifizierten Feinheit berücksichtigt wird. Eine solche Umrechnung eines Plans kann beispielsweise so erfolgen, wie auch die vorberechneten Pläne in der Datenbank für eine Vielzahl von möglichen Lösungen errechnet werden. Das zugehörige Verfahren ist öffentlich zugänglich und zu finden in Philipp Süss, A primal-dual barrier algorithm for the IMRT planning problem - An application for optimization-driven adaptive discretization, Mensch und Buch Verlag (mbv), Berlin, 2008.

Der so erstellte erste Navigationsplan ist in dem Sinne der Pläne/Lösungen aus dem vorberechneten Potential in dem Datenspeicher kein "neuer" Plan, sondern ein umgerechneter erster Plan. Nur der erste Plan kommt als ein Plan aus dem als Datenbank fungierenden Datenspeicher und wurde dargestellt, um daraus das Initialvoxel auszuwählen, an dessen Stelle oder um dessen Stelle herum eine lokale Dosisveränderung vorgenommen werden soll. Bleibt also der erste Plan weitgehend erhalten, spricht Anspruch 1 nur von einem Umrechnen dieses Plans.

Eine Umrechnung eines solchen Plans kann auf zumindest zwei verschiedene Arten erfolgen. Eine erste Umrechnung ist diejenige, mit dem Verfahren von Philipp Süss keine Gewichte vorzugeben. Eine zweite Umrechnungsvariante, mit der ein zweiter Navigationsplan erstellt werden kann, ist diejenige, mit mathematischen Gewichten zu arbeiten. Ein mathematisches Gewicht bei einer Umrechnung eines Ausgangsplans (des ersten Plans gemäß Anspruch 1) hat die Wirkung, dass mit zunehmendem Abstand von dem Initialvoxel diese mathematischen Gewichte steigen und die lokal begrenzte Änderung bei Vorhandensein von mathematischen Gewichten stärker begrenzt ist. Der genannte zweite Navigationsplan hat also gegenüber dem ersten Navigationsplan lokal begrenztere Änderungen. Bei dem ersten, ohne mathematische Gewichte berechneten Navigationsplan sind die Änderungen weiter gestreut.

Die mathematischen Gewichte können so gestaltet werden, dass sie über verschiedenen Richtungen vom Initialvoxel ausgehend nicht einheitlich, sondern in Abhängigkeit vom jeweiligen Gewebe gesetzt oder vorgegeben werden. Dies ist eine uneinheitliche, gewebeabhängige Gewichtung (Anspruch 7).

Die erste Bedienhilfe kann beispielweise eine Schiebeeinstellung oder eine Dreheinstellung sein (kurz: Schieberegler oder Drehregler), wobei an sich nicht geregelt, sondern nur verändert wird. Die erste Bedienhilfe hat zwei Endstellungen, wobei die erste Endstellung dem ersten Plan entspricht. Diese Entsprechung ist so, dass die Lage des Einstellknopfes der Bedienhilfe bestimmt, welcher Plan auf der Darstellungseinrichtung dargestellt wird. Liegt der Einstellknopf der ersten Bedienhilfe bei der ersten Endstellung, so wird der erste Plan dargestellt, von dem überhaupt ausgegangen wird.

Die zweite Endstellung der ersten Bedienhilfe kann auf verschiedene Weisen belegt sein.

Die zweite Endstellung kann dem umgerechneten ersten Plan entsprechen, der oben "erster Navigationsplan" genannt wird. Abhängig von der Stellung des Einstellknopfes (der Bedienhilfe) wird interpoliert oder mit der Einstellung der Bedienhilfe die Parameter einer zu erfolgenden Interpolation vorgegeben.

Zusätzlich zu diesen zwei genannten Endstellungen können mehrere Zwischenstellungen kommen. Jede Zwischenstellung entspricht einem Zwischenplan, wobei dieser Zwischenplan aus einer Interpolation folgt. Die Interpolation beginnt beim ersten Plan und geht in Richtung des ersten Navigationsplans (mit Bezug auf Anspruch 1). Je weiter die Bedienhilfe in Richtung zum ersten Navigationsplan, also der zweiten Endstellung verstellt wird, desto ähnlicher wird die Darstellung dem ersten Navigationsplan. Eine Interpolation ist dabei eine temporär errechnete Zwischenlösung (als Zwischenplan), die entsprechend der Stellung des Knopfes der Bedienhilfe weiter oder weniger weit vom ersten Plan entfernt ist, respektive inhaltlich näher oder weniger nahe dem ersten Navigationsplan ist.

Interpolierte Zwischenpläne sind nicht rechenintensiv und können relativ schnell berechnet und in einem Zwischenspeicher abgelegt werden, der sie für eine Verstellbewegung der Bedienhilfe erneut verfügbar macht.

All diese Umrechnungen betreffen oder berücksichtigen die Dosisänderung in der kleinen/lokalen Voxelgruppe und gehen grundsätzlich vom ersten Plan aus. Keiner dieser Zwischenpläne ist einer der vorberechneten, in der Datenbank vorgespeicherten Lösungen, sondern stammen aus dem ersten Plan und den - über die Vorschriften von Philipp Süss errechneten - beiden Navigationsplänen. Die Umrechnung in die Navigationspläne beruht auf einer mathematischen Beschreibung ihrer Ziele, d.h. der lokalen Verbesserung in der kleinen Voxelgruppe bei lediglich geringfügigen Veränderungen an anderer Stelle. Das von Süss beschriebene Verfahren ermöglicht die Umrechnung mit einer solchen (Ziel-) Beschreibung.

Mit Hilfe dieses zumindest einen umgerechneten Navigationsplans und der zumindest einen ersten Bedienhilfe können Zwischenpläne generiert werden, die es dem Benutzer erlauben, quasi in einem Kontinuum zu steuern und eine Vielzahl von Zwischenplänen zu sichten, darzustellen und zu bewerten, ob sie das gewünschte Ziel hinreichend erreichen, namentlich die Dosisänderung in der kleinen Gruppe von Voxeln, die bei Beibehaltung des Planes im Wesentlichen (nicht ganz, aber sehr ähnlich) und im Wesentlichen derselben DVH-Verteilung als lokal begrenzte Änderung durchzuführen ist.

Wie bereits beschrieben, gibt es Zwischenstellungen der in ihrer Einstellung veränderbaren Bedienhilfe. Es erfolgt ein Darstellen eines Zwischenplans auf derselben Darstellungs-Einrichtung, abhängig von der veränderbaren Einstellung der ersten Bedienhilfe. Sie hat zwei Endstellungen und mehrere Zwischenstellungen. Die erste Endstellung entspricht nach wie vor dem ersten Plan (dem Eingangsplan) und jede der Zwischenstellung entspricht einem andern Zwischenplan. Diese werden jeder aus einer jeweiligen Interpolation zwischen dem ersten Plan und dem Navigationsplan gewonnen. Einer der interpolierten Zwischenpläne ist zur (späteren) Einstellung am technischen Gerät der kausale Folgeplans, mit dem die Änderung des ersten Plans in der volumenmäßig spezifizierten Feinheit erfolgte.

Die Zwischenstellungen selbst brauchen nicht gerastert zu sein, sondern können quasi-kontinuierlich erfolgen, wobei die Länge der Bedienhilfe oder der Drehwinkel der Bedienhilfe eine Rolle spielt und die Diskretisierung bestimmt, wie viele Zwischenpläne auf der Stelllänge oder dem Stellwinkel unterzubringen sind, so dass der Benutzer eine Art kontinuierliches Gefühl erhält, obwohl er einzelne interpolierte Zwischenpläne bei der Drehung oder dem Schieben dargestellt erhält.

Mit der weiteren Bedienhilfe kann vorgegeben werden, welcher Endwert der ersten Bedienhilfe eingestellt wird. Ist die zweite Bedienhilfe nur ein Schalter, kann zwischen dem ersten Navigationsplan und dem zweiten Navigationsplan umgeschaltet werden. Ist die zweite Bedienhilfe auch ein Stellglied, beispielsweise ein Schieberegler oder ein Drehregler, so können mehrere verschiedene Pläne als Endwert der ersten Bedienhilfe definiert werden, die wiederum als Interpolation umgerechnet werden, zwischen dem ersten Navigationsplan und dem zweiten Navigationsplan. So ergibt sich der dritte Navigationsplan als Endwert der ersten Bedienhilfe, vorgegeben oder vorgesteuert von der Stellung der zweiten Bedienhilfe.

In den Ausführungsbeispielen werden zwei Slider (geradlinige Einstelleinrichtungen) dargestellt. Es sind ebenso Drehregler möglich oder sogar flächige Einstellglieder, bei denen aus den beiden linearen Bedienhilfen eine Fläche definiert wird, beispielsweise in Form eines Dreiecks. Innerhalb der Fläche des Dreiecks kann quasi kontinuierlich verändert werden.

Diese flächige Ausbildung einer Bedienhilfe wird zumindest erfasst von Anspruch 1, wobei zwei Ecken des Dreiecks die beiden Endstellungen der ersten Bedienhilfe darstellen. Liegt die zweite Bedienhilfe als beispielsweise lineares Streckenstück senkrecht zur ersten Bedienhilfe, bildet sie mit diesen durch die Endpunkte ein Dreieck, und eine Position, welche beispielsweise ein Cursor abweichend von der geraden Linie der ersten Bedieneinrichtung (zwischen den beiden ersten Ecken des Dreiecks) hin zum dritten Eck einnimmt ist ein Maß dafür, wie die zweite Bedieneinrichtung den Endwert der ersten Bedieneinrichtung verändert.

Das Dreieck funktioniert alternativ auch für den speziellen Fall, dass genau drei Pläne interpoliert werden. Diese drei Pläne sind den Ecken zugeordnet werden, und die Positionierung der Bedienhilfe innerhalb der von diesen Ecken aufgespannten Dreiecksfläche entspricht einer Interpolation zwischen diesen drei Plänen.

Diese Bedienung hat eine gewisse Ähnlichkeit mit Anspruch 1 dahingehend, dass die drei Pläne unabhängig von ihrer Herkunft (fest) vorgegeben sind und dazwischen interpoliert wird.

Für den Benutzer ergibt sich auch bei Verwenden nur einer Bedienhilfe eine Verbesserung der Feinheiten der Veränderung, die eine nur lokal begrenzte Veränderung zum Ziel hat. Der Benutzer kann sich durch Stellung der ersten Bedienhilfe oder ggf. durch Hinzunahme der Stellung der zweiten Bedienhilfe in einem Kontinuum von Plänen bewegen, die alle auf den ersten Plan zurückgehen und während der Bewegung der Bedienelemente interpoliert werden. Eine solche Interpolation bringt es mit sich, dass eine Einstellung der ersten Bedienhilfe näher zur ersten Endstellung einem Zwischenplan entspricht, der näher dem ersten Plan ist.

Entsprechendes gilt auch für die Nähe eines Stellknopfes der ersten Bedienhilfe mit Bezug auf die zweite Endstellung, nur ist der dargestellte Zwischenplan dabei näher dem ersten Navigationsplan oder einer Mischung aus zwei weiteren Navigationsplänen, die beispielsweise mit und ohne mathematischem Gewicht umgerechnet wurden oder aber die zweite Endstellung entspricht dem zweiten Navigationsplan, wenn eine zweite Bedieneinrichtung es so vorgibt und die zweite Bedieneinrichtung zwischen dem ersten und dem zweiten Navigationsplan oder einer Mischung daraus verändern kann.

Als Interpolation kann beispielsweise eine Berechnung verwendet werden, die aus dem ersten Plan, abhängig von der Lage des Einstellknopfes der ersten Bedienhilfe einen Zwischenplan errechnet, der zwischen dem ersten Plan und dem ersten Navigationsplan liegt. In gleicher Weise kann diese Interpolation auch zwischen dem ersten und zweiten Navigationsplan ausgeführt werden, wobei der erste Navigationsplan ohne mathematische Gewichte und der zweite Navigationsplan mit mathematischen Gewichten aus dem ersten Plan über die Berechnungsvorschrift von Süss umgerechnet wird und abhängig von der Position der zweiten Bedienhilfe diese Interpolation mehr zum ersten Navigationsplan oder mehr zum zweiten Navigationsplan hin verändert, um den dritten Navigationsplan zu definieren, der Endwert der ersten Bedienhilfe wird.

Die Interpolation kann wie folgt ausgebildet sein.

Interpolation ist die Generierung eines in der Regel transienten Zwischenplans aus existierenden Navigationsplänen. Dem Namen entsprechend liegt dieser interpolierte Zwischenplan zwischen den zu seiner Generierung benutzten Plänen.

Die am häufigsten benutzte Methode ist die so genannte Konvexkombination. Hier wird eine Reihe von Ausgangsplänen mit zugehörigen Gewichten entsprechend der Gewichte zusammen gemischt. Die Gewichte summieren sich dabei zu 100%. Das Zusammenmischen wird auf die Fluenzen der Pläne (physikalische Planparameter) angewendet. Da die Strahlendosis (in sehr guter Näherung) linear von den Fluenzen abhängt, können auch die Dosisverteilungen der Pläne analog zu den Fluenzen gewichtet zusammenaddiert werden.

Da jedoch die Fluenzen nicht die Einstellungen des Bestrahlungsgeräts sind, sondern dort die Blattstellungen des Multileaf-Kollimators mit zugehörigen Zeiten primäre Größen sind, werden die Fluenzen (die deshalb theoretisch genannt werden) in einem "Sequencing" genannten Prozess in solche Blattstellungen umgerechnet. Die Umwandlung der Geräteeinstellungen geschieht also nicht mittels Konvexkombination.

Außer Konvexkombination wären auch etwas kompliziertere Interpolationsmechanismen möglich. Zum Beispiel könnten die Pläne dabei auch skaliert werden.

Die Interpolationsparameter (z.B. welche Pläne, welche Gewichte) können direkt aus der Stellung der Bedienhilfe abgelesen werden oder auch indirekt aus der Stellung abgeleitet werden. Es wird ein Qualitätswert (nicht ein Parameterwert!) mit der Stellung der Bedienhilfe eingestellt und in einem sehr kleinen Optimierungsproblem die Interpolationsparameter so bestimmt, dass sich die Werte der anderen Achsen möglichst wenig ändern. Dieses Problem ist so klein, dass es in Echtzeit gelöst werden kann, so dass dem Benutzer die Interpolation nicht bewusst ist.

Es versteht sich, dass die Mittenstellung der Bedienhilfe einen solchen Zwischenplan auf der Darstellungseinrichtung zur Anzeige veranlasst, kurz: anzeigt, der interpoliert zwischen dem ersten Plan und dem ersten Navigationsplan oder dem dritten Navigationsplan liegt, wobei der dritte Navigationsplan der erste, der zweite oder eine Mischung daraus sein kann (Anspruch 2).

Es sollte nochmals erwähnt sein, dass die Pläne keine reine Information sind, sondern technische Daten und damit Dosisverteilungen entsprechen, die in dem technischen Gerät der Tumortherapie eingestellt werden oder entsprechen (Anspruch 3).

Es ist praktisch unmöglich, diese technischen Geräteeinstellungen in einer Weise verständlich für den Benutzer in einem Patentanspruch niederzulegen, ohne auf eine Darstellung Bezug zu nehmen, die für den Betrachter verständlich ist. Eine Kolonne von Bestrahlungsintensitäten, Bestrahlungszeiten und zugehörigen Winkelangaben ist für den Benutzer keine wahrnehmbare oder bewertbare Geräteeinstellung. Sie ist eine reine technische Einstellung, die von einer übergeordneten Stelle heraus errechnet oder berechnet wird, welche übergeordnete Stelle für den Betrachter mit dem Therapieplan in verständlich Weise zusammenhängt. So die Schnitte mit den Isodosis-Linien, die Beispiele sind, oder ein DVH-Diagramm, welches hilfreiche, aber nicht ausreichende Repräsentanz für den gesamten Plan in einem Bild als Bewertungsmaßstab gibt.

Die interpolierten Zwischenpläne, die während der Bewegung der Bedienhilfe (der ersten oder der zweiten Bedienhilfe) entstehen, werden in der Datenbank nicht gespeichert. Sie sind temporäre Zwischenpläne, die keine der vorberechneten Pläne sind. Sie sind diesen vorberechneten Plänen zu ähnlich. Sie werden aber abgespeichert in einem Zwischenspeicher, um sie bei Bedarf, d.h. bei Gefallen des Nutzers, der diesen interpolierten Plan für seine Zwecke gut findet, zu exportieren und für die Geräteeinstellung bereitzustellen. Dieser exportierte Plan ist der kausale Folgeplan, der erstellt oder ermittelt werden soll und zu dessen Zweck die oben umschriebenen Schritte der Erfindung erfolgen. Eine Datenbank ist in diesem Sinne auch ein Speicher, aber nicht derjenige Speicher, für den die interpolierten (flüchtigen) Pläne vorgesehen sind (Anspruch 4).

Es ist zuvor von Bedienhilfen gesprochen worden, von Cursor und von Drehreglern oder Schiebereglern. Diese sind physisch nicht zwingend als greifbare Dreh-Einsteller zu versehen, wie beispielsweise ein Potentiometer oder ein Schieberegler. Diese Darstellungen werden in den Anwendungen zumeist optisch auf einem Bildschirm dargestellt und können hierbei auf einer gesonderten Darstellungseinrichtung dargestellt sein, während die andere Darstellungseinrichtung die Plandarstellung übernimmt, aus beispielsweise einem DVH-Diagramm und drei Schnittdiagrammen mit Isodosis-Linien (transversal, sagital und frontal).

Es können weitere Anzeigegrößen hinzutreten, die zusätzliche Ähnlichkeiten oder sonstige Änderungswerte repräsentieren, und diese können entweder auf der ersten oder zweiten Darstellungseinrichtung Platz finden.

Wenn so gewünscht, können die erste oder/und die zweite Bedienhilfe aber auch physisch herausgeführt werden und als anfassbare Potentiometer dem Benutzer an die Hand gegeben werden, der dann physisch drehend oder schiebend (ohne Verwendung eines Zeigergerätes mit Cursor) eine Planveränderung am Bildschirm vornimmt. Die Umsetzung über einen AD-Wandler oder über ein gerastertes Potentiometer mit diskreten Stellungen sind hier nur zwei von vielen Möglichkeiten, dem Benutzer die Bedienführung des Systems so einfach und praktisch günstig wie möglich zu gestalten.

Einen Einstellknopf kann man wirkungsmäßig gleich bildlich auf einer Darstellungseinrichtung darstellen und mit einem Zeiger einer Steuereinrichtung (ein Mausgerät) betätigen, oder aber physisch herausführen und greifbar für den Benutzer zum Anfassen zur Verfügung stellen.

Ausführungs**beispiele** der Erfindung werden anhand von Figuren näher erläutert. Hierzu eine Übersicht.
- Figur 1: ist ein Blockschaltbild einer Steueranordnung mit zwei Darstellungseinrichtungen 110, 111.
- Figur 2: ist symbolisch das Planvolumen Z, welches in der folgenden Figuren 4 gezeigt ist.
- Figur 3: ist ein DVH Diagramm 70.
- Figur 4a, Figur 4b und Figur 4c: veranschaulichen jeweils eine Schnittebene (ein Slice) in drei Raumrichtungen.
- Figur 5: ist eine Zusammenfassung der Einzeldarstellungen der Figuren 3, 4a, 4b, 4c und ihren Teilbildern 70, 72, 74, 76 mit Hinzunahme von Bedienhilfen 30, 40.
- Figur 6: zeigt die Auswahl eines Initialvoxels z1.
- Figur 7: zeigt den Dosiswert an der ausgewählten Stelle z1 von Figur 6 in einem eingeblendeten Fenster 77.
- Figur 8: zeigt ein weiteres Fenster 78, welches nach Bestätigung des Fensters aus Figur 7 eingeblendet wird.
- Figur 9: zeigt aktivierte Bedienhilfen 30, 40, die nach Vorhandensein eines ersten Navigationsplans und/oder eines zweiten Navigationsplans aktiviert werden.
- Figur 10: zeigt eine der Figur 9 entsprechende Darstellung.
- Figur 11: veranschaulicht eine andere Einstellung der Bedienhilfen 30, 40.
- Figur 12: veranschaulicht die Erstellung des kausalen Folgeplans 20.
- Figur 13: veranschaulicht die Erstellung eines weiteren kausalen Folgeplans 21.
- Figur 14: veranschaulicht ein DVH Schaubild 70".

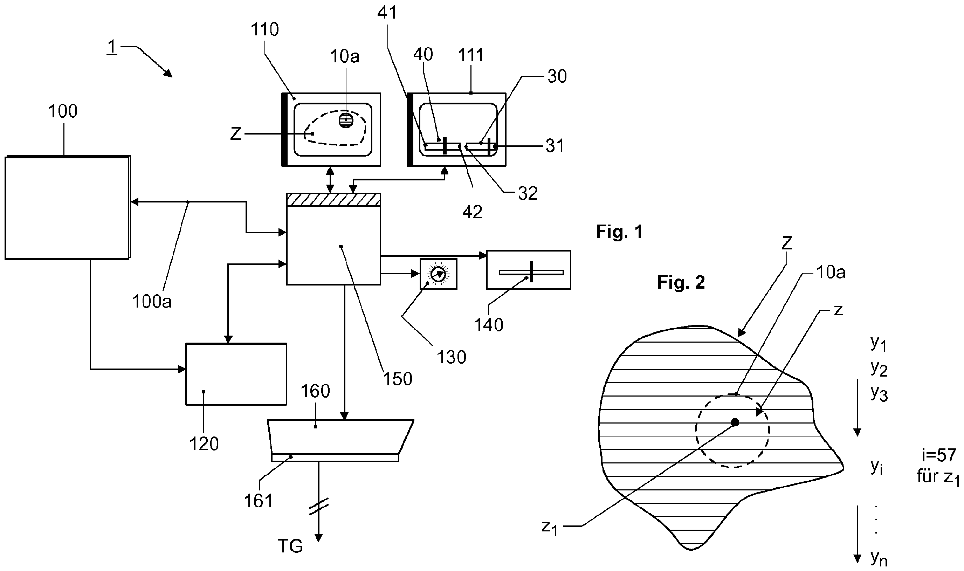

**Figur 1** ist ein Blockschaltbild einer Steueranordnung mit zwei Darstellungseinrichtungen 110, 111. 100 ist eine Datenbank und 150 ein zentraler Steuer- und Rechenkern mit einem Zwischenspeicher 120, der zur Ablage und Zwischenspeicherung von interpolierten Plänen dient.

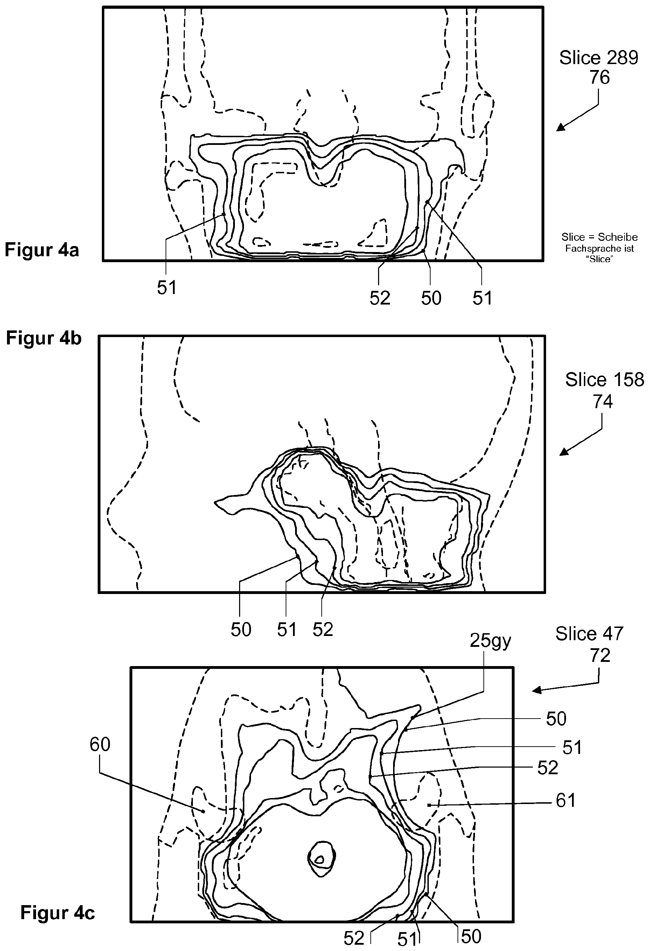

**Figur 2** ist symbolisch das Planvolumen Z, welches in den folgenden Figuren 4 gezeigt ist. Es ist hier eine Ebene dargestellt, von denen es viele Ebenen gibt, bezeichnet mit y₁ bis yₙ, wobei in der Ebene yᵢ das Initialvoxel z1 zu liegen kommt, wie später ausgewählt und später erläutert. Z repräsentiert das in mehrere Schichten in drei Raumrichtungen zur Berechnung und Darstellung aufgeteilte Planvolumen Z, innerhalb dessen das lokal begrenzte, Volumen z liegt, welches die volumenmäßig spezifizierte Feinheit mit ihrer Grenze 10a darstellt.

**Figur 3** ist ein DVH-Diagramm 70 mit einem Volumenanteil als vertikale Achse und einer Dosisskala zwischen 0 gy und 100 gy. Aufgetragen als Kennlinien sind sowohl Zielvolumen (erster und zweiter Tumor, ganz rechts, Bezugszeichen 80 und 81), wie auch Kennlinien (Kurven) von Risiken, die im Folgenden näher erläutert werden.

**Figur 4a, Figur 4b und Figur 4c** veranschaulichen jeweils eine Schnittebene (ein Slice) in drei Raumrichtungen, wobei Figur 4a die Schnittebene 289 der frontalen Schnitte zeigt, Figur 4b die Schnittebene 234 der sagitalen Darstellung und Figur 4c die Schnittebene 57 der transversalen Schnittebenen zeigt. Die Schnittebenen werden oft auch "Slice" genannt, wobei das Planvolumen in drei Raumrichtungen in eine bestimmte Anzahl von Slices je Raumrichtung aufgeteilt wird und innerhalb eines jeweiligen Slices werden Isodosis-Linien definiert, ähnlich den Höhenlinien eines Gebirgszugs, wobei diese Isodosis-Linien die Linien gleicher Dosis charakterisieren. So ist in Figur 4c eine Stufung der Dosen von 25gy, 33gy, 40gy, 50gy, 60gy und 65gy, also sechs Stufen von Dosen dargestellt. In einem anschaulichen Beispiel ist die äußere Linie die Isodosis-Linie von 25gy und es sind zwei Organe 60, 61 räumlich repräsentiert, die links und rechts Nierenform als linke und rechte Ohrspeicheldrüse darstellen. Die Isodosis-Linien 50, 51, 52 sind in den Beispielen für drei außen liegende Isodosis-Linien beziffert.

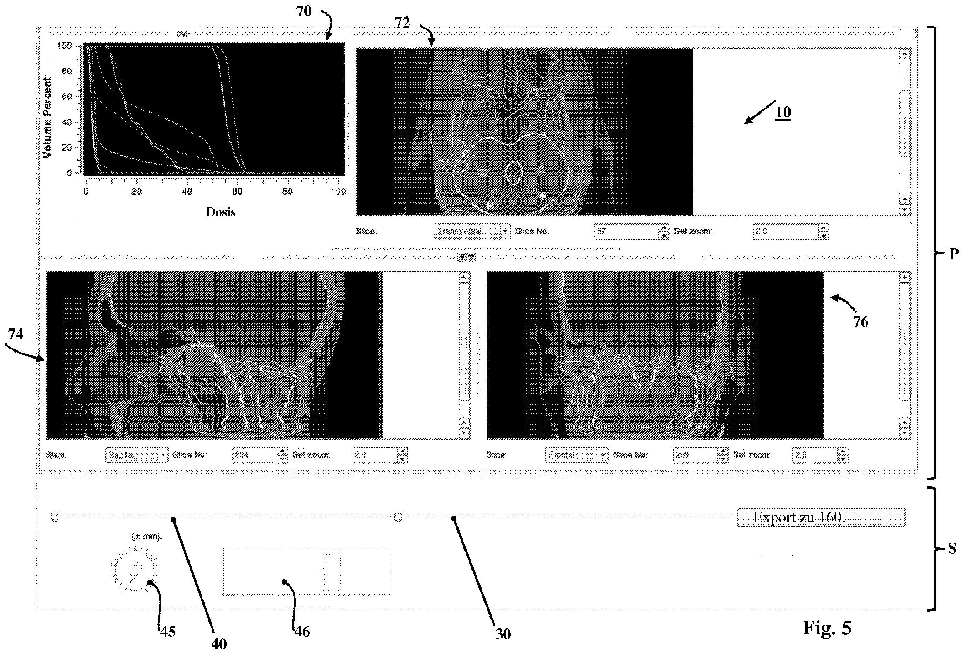

**Figur 5** ist eine Zusammenfassung der Einzeldarstellungen der Figuren 3, 4a, 4b, 4c und ihren Teilbildern 70, 72, 74, 76 uns mit Hinzunahme von Bedienhilfen 30, 40. Die vier Darstellungen aus DVH-Diagramm und drei Schnittebenen von Isodosis-Linien repräsentieren "einen Plan".

In Figur 5 sind im Teilbild 70 mehrere Kennlinien von Organen, Gewebebereichen und Zielvolumen gezeigt. Die beiden rechten Kennlinien sind zwei Zielvolumen (Tumorvolumen). Die mittlere Kurve mit der langen Schräge repräsentiert den Hirnstock. Die beiden steiler und im Wesentlichen parallel verlaufenden Kennlinien sind die linke und die rechte Ohrspeicheldrüse. Die flacher verlaufende Kurve, die im Mittelabschnitt ungefähr parallel zur Kennlinie des Hirnstocks verläuft, ist der Body. Die einer 1/x Funktion angenäherte Kurve ist die Dosiskennlinie für das gesamte Gehirn und die übrigen, steil abfallenden Kurven sind Risikoorgane (Optik, linkes und rechtes Auge).

Die Bedienhilfen 30, 40 im Einstellabschnitt S sind auf derselben Darstellungseinrichtung, beispielsweise derjenigen 110 von Figur 1, zusammen mit der Plandarstellung sichtbar. Der untere Abschnitt S, welcher die Einstellungshilfen beinhaltet, könnte auch auf der Darstellungs-Einrichtung 111 Platz finden, so dass nur der Abschnitt P für die Repräsentierung des eingestellten Plans auf der Darstellungs-Einrichtung 110 verbleibt. Ebenso könnte die Einstellsektion S durch eine Hardware realisiert werden, z.B. mit zwei herausgeführten Einstellreglern, die manuell betätigbar sind, wie diejenigen 130, 140 aus Figur 1. Figur 5 stellt den ersten Plan 10 dar, der gleichermaßen Repräsentant für eine Vielzahl technischer Einstellparameter des nicht dargestellten Gerätes TG der Tumortherapie ist, vgl. z.B. US 6,038,283 A (Carol et al, Nomos), dort Fig. 1.

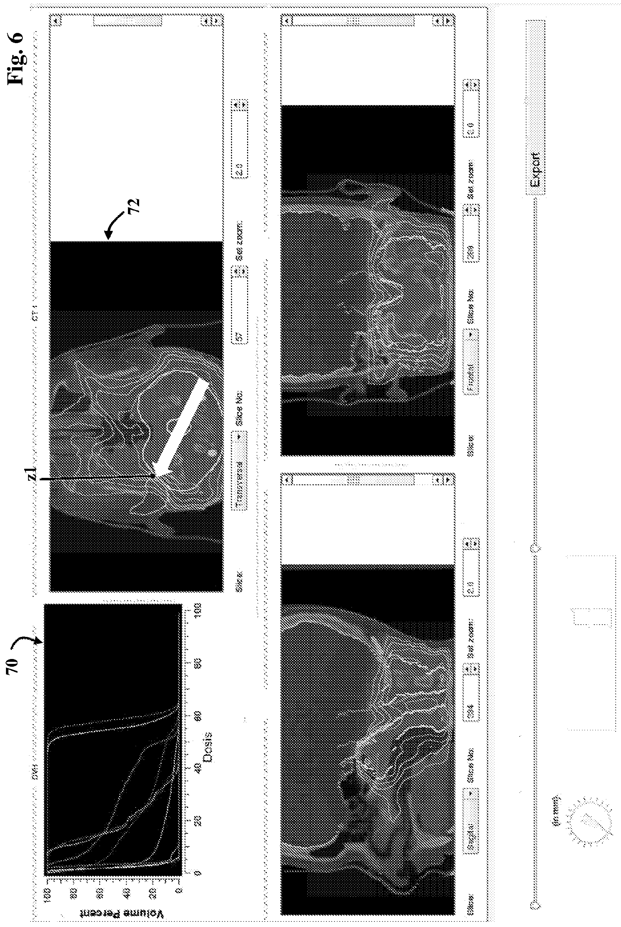

**Figur 6** zeigt die Auswahl eines Initialvoxels z1. Dieses befindet sich in Schicht 57 der transversalen Schnitte (oberes rechtes Diagramm). Die anderen Darstellungen haben sich gegenüber Figur 5 nicht verändert, da nur eine Auswahlaktion mit einem Mauszeiger erfolgte.

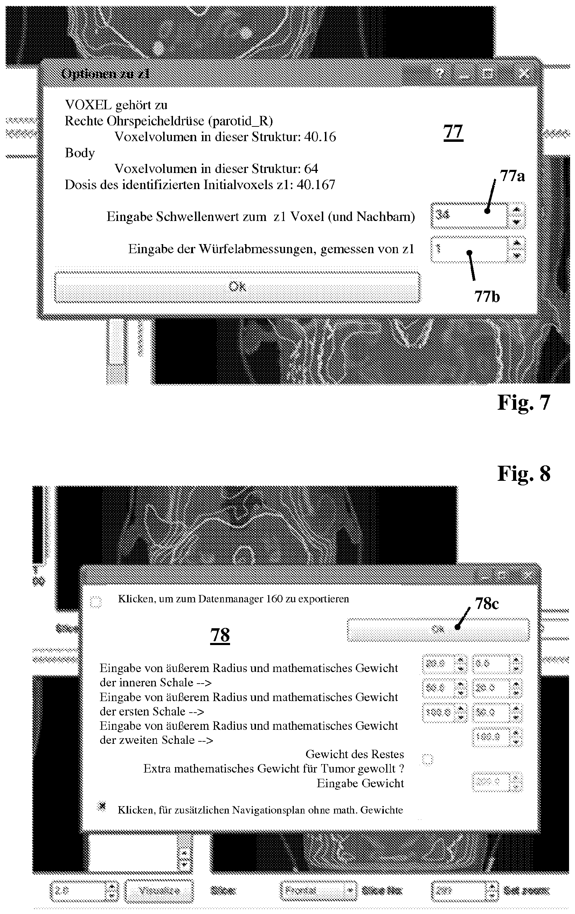

**Figur 7** zeigt den Dosiswert an der ausgewählten Stelle z1 von Figur 6 in einem eingeblendeten Fenster 77 mit Informationen zum Initialvoxel und mit Einstellmöglichkeiten zur Größe der volumenmäßig spezifizierten Feinheit und der Wunschdosis.

**Figur 8** zeigt ein weiteres Fenster 78, welches nach Bestätigung des Fensters aus Figur 7 eingeblendet wird. Hier werden Parameter zur Umrechnung eingestellt, mit denen der erste und zweite Navigationsplan erstellt, bzw. aus dem ersten Plan, der in Figur 5 dargestellt ist, umgerechnet werden.

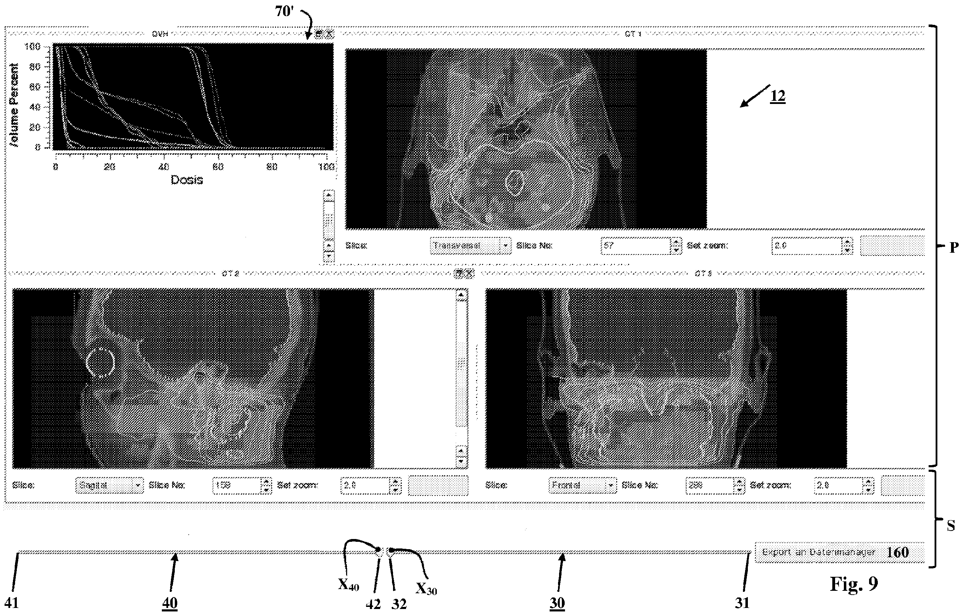

**Figur 9** zeigt aktivierte Bedienhilfen 30, 40, die nach Vorhandensein eines ersten Navigationsplans und/oder eines zweiten Navigationsplans aktiviert werden. Die Stellknöpfe X40 und X30 der beiden als Schiebeeinsteller 40, 30 dargestellten Bedienhilfen können zwischen links und rechts verschoben werden und haben auch Zwischenstellungen. Jede der "Slider" (Schieber) hat einen linken und einen rechten Anschlag, der Endanschlag oder Endstellung genannt wird. Die Endanschläge 41, 42 zur Bedienhilfe 40 und 31, 32 zur Bedienhilfe 30 sind in Figur 9 repräsentiert. Dargestellt wird der zweite Navigationsplan 12 in dem oberen Planabschnitt P der Darstellungseinrichtung, die auch vollständig diejenige 110 der Figur 1 sein könnte, wohingegen der Einstellabschnitt S auch auf der zweiten Darstellungs-Einrichtung 111 platziert sein könnte, im Bild der Figur 9 aber beide gemeinsam auf einer Darstellungseinrichtung vorhanden sind.

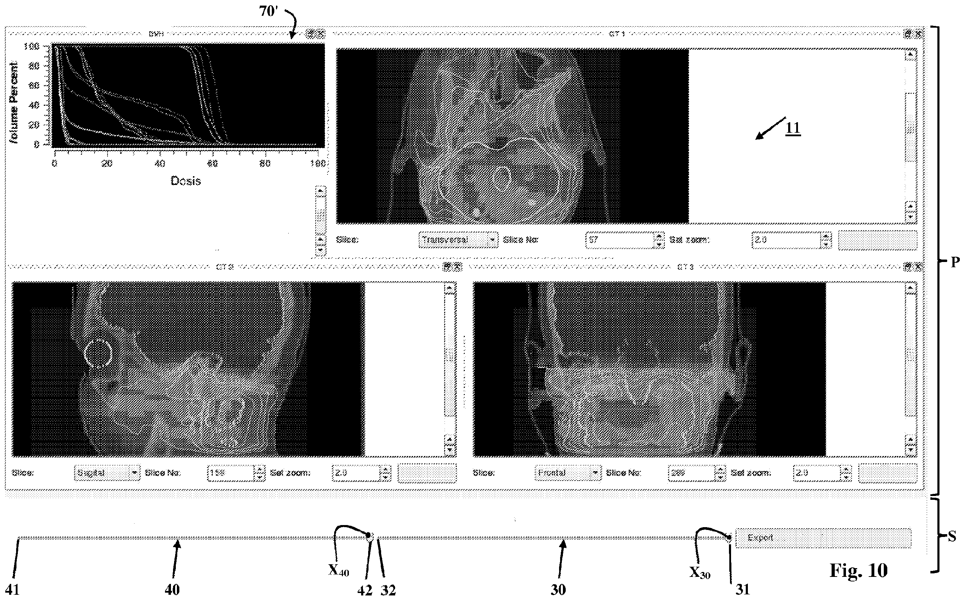

**Figur 10** zeigt eine der Figur 9 entsprechende Darstellung, nur ist hier im Planabschnitt P der erste Navigationsplan 11 dargestellt. Dies aufgrund der Einstellung der Schiebeknöpfe X30, X40 der beiden Bedienhilfen 30, 40, wie später vertieft erläutert wird.

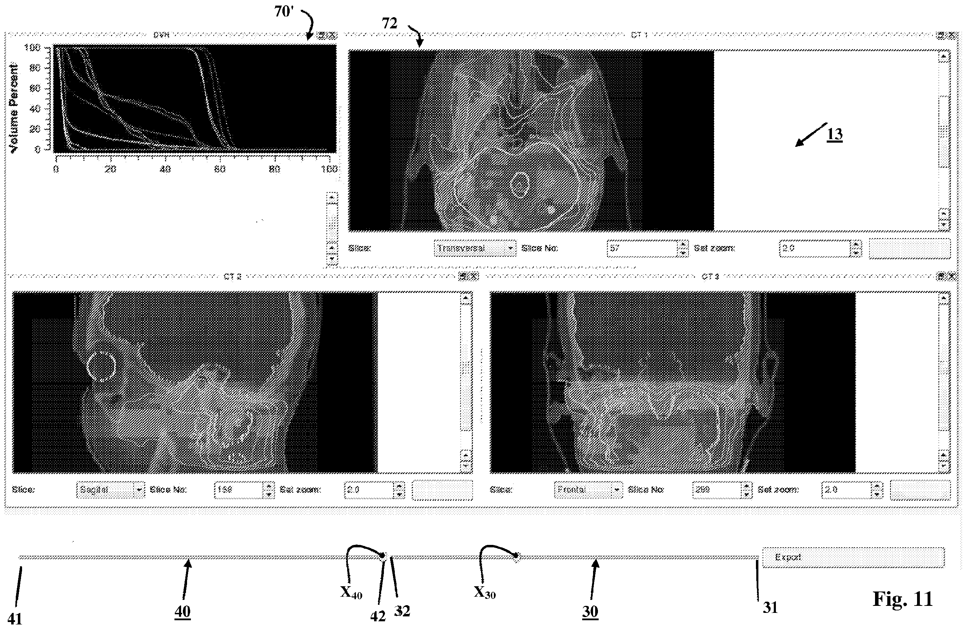

**Figur 11** veranschaulicht eine andere Einstellung der Bedienhilfen 30, 40. Hier ist die erste Bedienhilfe 40 auf der linken Seite an ihrem rechten Anschlag 42 und stellt den Plan im Planabschnitt P dar, welche von der rechten Bedienhilfe 30 vorgegeben wird. Deren Einstellknopf X30 steht in einem Zwischenbereich an der Stelle 33 und repräsentiert einen Zwischenplan zwischen den Stellungen 31 und 32, der eine Interpolation darstellt zwischen dem ersten Navigationsplan 11, der auf Position 31 angewählt wird und dem zweiten Navigationsplan 12, der auf Position 32 angewählt wird. Es ergibt sich an der Position 33 ein dritter Navigationsplan 13, der den Endwert der ersten Bedienhilfe 40 vorgibt, und wenn deren Einstellknopf X40 an der zweiten Endstellung platziert ist, wie in Figur 11 dargestellt, wird im Planabschnitt P der dritte Navigationsplan 13 dargestellt.

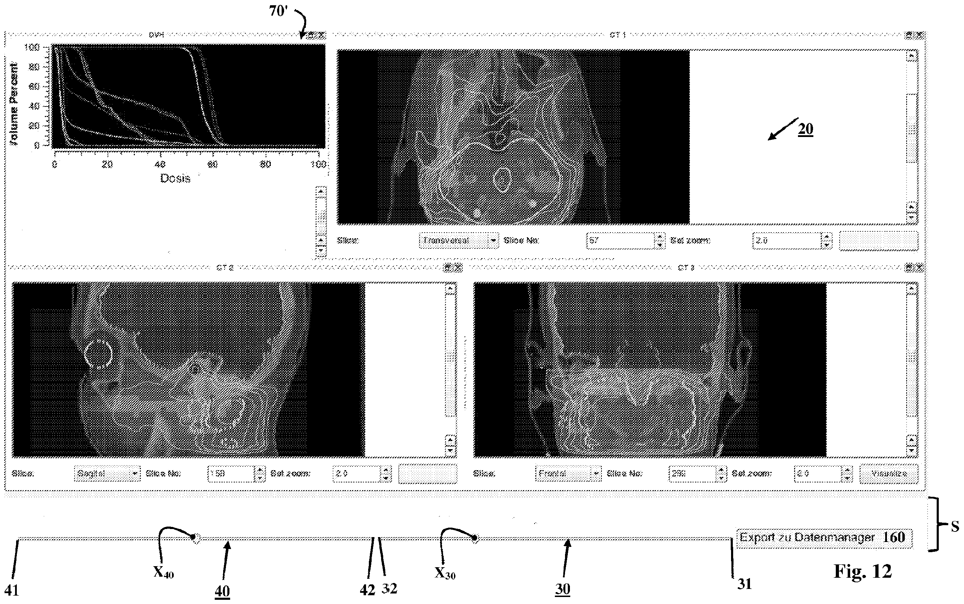

**Figur 12** veranschaulicht die Erstellung des kausalen Folgeplans 20, wie er durch Zwischenstellungen 33, 43 der ersten und zweiten Bedienhilfe 40, 30 zustande kommt, und zwar jeweils interpoliert, wie weiter unten vertieft erläutert wird.

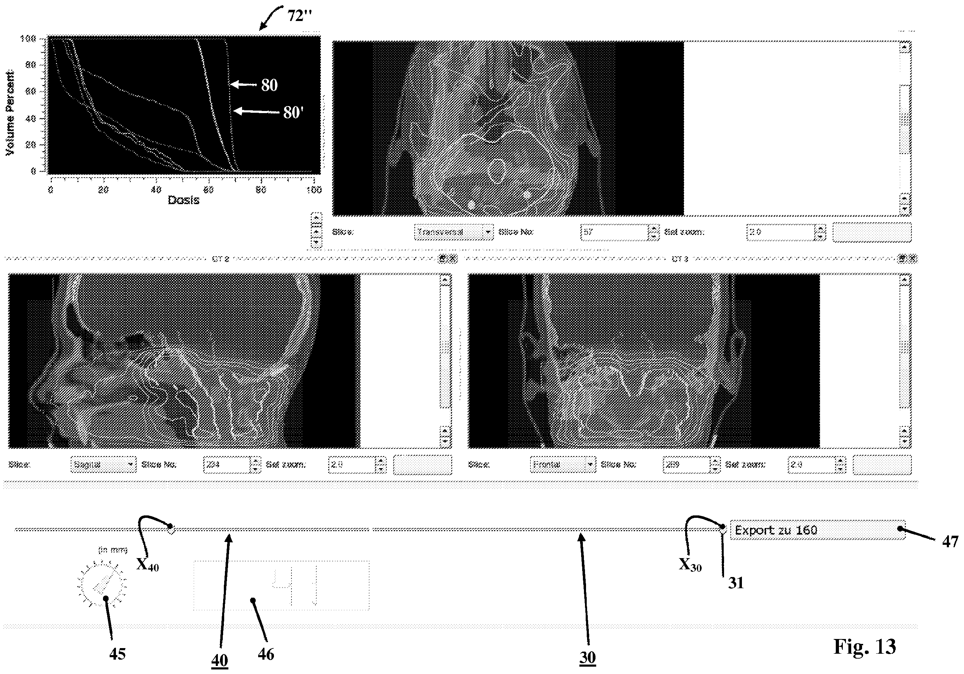

**Figur 13** veranschaulicht die Erstellung eines weiteren kausalen Folgeplans 21, wie er durch eine andere Zwischenstellung der ersten und zweiten Bedienhilfe 40, 30 zustande kommt, und zwar jeweils interpoliert, wie weiter unten vertieft erläutert wird.

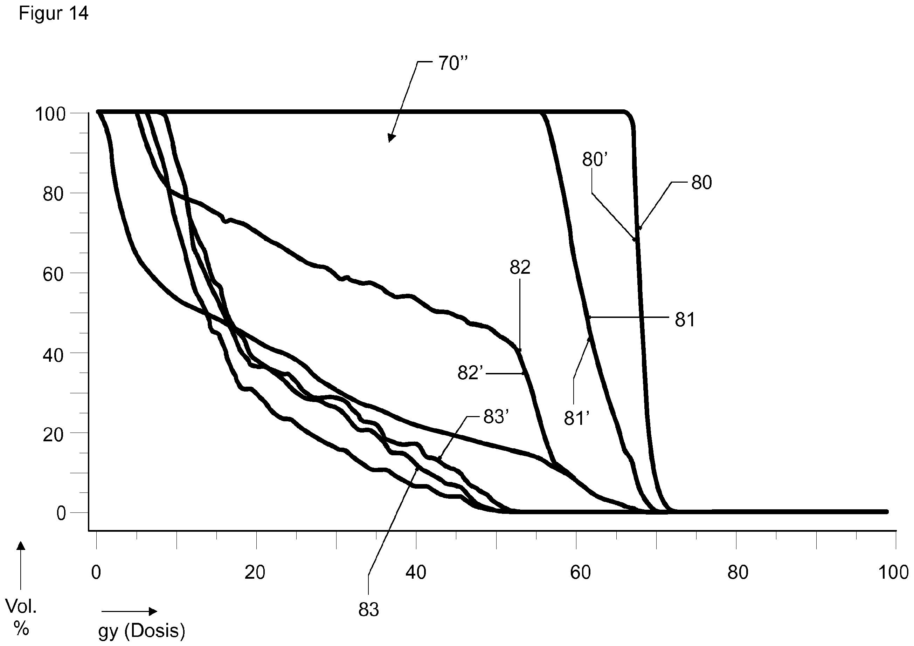

**Figur 14** veranschaulicht ein DVH Schaubild 70", welches beide Pläne, den ersten Plan 10 und den kausalen Folgeplan 21 von Figur 13 in einem Bild, das in zwei Ansichten dargestellt ist, repräsentiert. Es sind wenige Kurven als Kennlinien herausgegriffen, aber immer als Paar dargestellt, z.B. das Tumorgebiet mit den Kennlinien 80 und 80'.

In den vorgenannten erweiterten Übersichten zu den Figuren war jeweils ein Plan im Planabschnitt P dargestellt. Dieser Plan besteht im Beispiel aus vier Abbildungen 70 bis 76, die mit den Figuren 3, 4a, 4b und 4c erläutert werden. Es sind ein DVH-Diagramm 70 und drei Schnittdiagramme, wobei zu jedem Bild (Segment) die Schnittebene angegeben ist, respektive die Slice-Nummer, die für die Erläuterung durchgehend gleich gehalten wurde.

Der transversale Schnitt liegt bei Slice 57, der sagitale Schnitt bei Slice 158 und der frontale Schnitt bei Slice 289. Dies gilt für alle Figuren, so dass ein Vergleich der Isodosis-Linien in den Darstellungen der Ausschnittbilder 72, 74 und 76 sowie der Isodosis-Darstellung des Bildes 70 zwischen allen Bildern möglich ist.

In den Darstellungen des Planbereichs oder der Planbereiche der Figuren 9 bis 13 wird das Bild 70 der DVH-Darstellung aus Figur 3 doppelt verwendet, und zwar zur Darstellung des ursprünglichen Plans 10 (an der Anfangsstellung 41 der ersten Bedienhilfe 40 gelegen) und dem durch Verändern der Position des Einstellknopfes X40 und/oder X30 erzielten neuen, geänderten Plan.

In dem DVH-Bild 70' ist dann für jede Kurve der Figur 3 eine weitere Kurve hinzugekommen, welche die Änderung repräsentiert, aber gleichermaßen ist erkennbar, dass diese Änderung so gering ist, dass der ursprüngliche Plan 10 (der Eingangsplan) aus Figur 5 praktisch noch vorhanden ist, nur mit einer neuen Maßgabe, die anhand der Figur 6 und der Einstellung der Figuren 7, 8 vorgenommen wurde.

Eine Übersicht über die Schaltungsstruktur und die Komponenten des Systems zur Auffindung eines kausalen Folgeplans, mit dem eine Einstellung eines technischen Geräts oder am technischen Gerät der Tumortherapie vorgenommen wird, ist in Figur 1 gezeigt.

Figur 1 ist in Verbindung mit Figur 5 zu lesen, da die Darstellungs-Einrichtung 110 von Figur 1 zumindest den Planabschnitt P von Figur 5 dem Benutzer visuell darstellt. Die einzelnen Segmente 70, 72, 74, 76 sind aus den Figuren 3, 4a, 4b, und 4c zu ersehen und dort näher erläutert. Sie repräsentieren einen Plan, der im Bild der Figur 5 der erste Plan 10 ist, der aus einer vorgespeicherten Menge von vorberechneten Plänen in der Datenbank 100 stammt.

Der Stellabschnitt S aus Figur 5, im Unterschied zum Planabschnitt P und unterhalb dieses Planabschnitts in Figur 5 dargestellt, kann auch gesondert auf einer weiteren Darstellungseinrichtung 111 platziert werden, die auch ein Bildschirm sein kann. Es kann aber ebenso ein mechanisches Stellglied Verwendung finden und die Bedienhilfen 30, 40 aus Figur 5 werden mechanische Stellglieder, als Potentiometer 130 oder Schieberegler 140 in Figur 1 repräsentiert. Diese sind alternativ zu den optisch dargestellten Stellgliedern, beispielsweise den Slidern 30,40 als englischsprachiges Synonym zu den Schiebereglern, vorhanden.

Die Bedienhilfen 30, 40 oder 130, 140 werden von einem nicht dargestellten Nutzer verwendet, um die Darstellung auf dem Schirm als Beispiel einer Darstellungseinrichtung 110 zu verändern. Er möchte dabei einen volumenmäßig spezifizierten feinen Bereich verändern, dessen Volumenrand mit 10a in Figur 2 repräsentiert ist. Schematisch ist auch das Planvolumen Z in Figur 2 gezeigt, welches realitätsnäher aus Figur 5 hervorgeht.

Dazu werden die Einstellhilfen 30, 40 zwischen ihren Endstellungen 31 und 32 sowie 41 und 42 bewegt. Entsprechendes kann auch mit den haptisch berührungsmäßigen Einstellern, die mit der Hand berührungsmäßig verändert werden, vorgenommen werden. Ein Touchscreen ist eine weitere Variante

Der zentrale Steuerkern 150 verbindet die genannten Komponenten funktionstechnisch und datentechnisch. Er greift auf die Datenbank 100 zu, bedient über eine Videoschnittstelle die Darstellungs-Einrichtung 110 und ggf. 111, nimmt Signale von den Bedienhilfen 30, 40 oder 130, 140 entgegen und hat einen Zwischenspeicher 120, in den interpolierte Zwischenpläne abgelegt und wieder abgerufen werden können.

Der Normalfall der Kopplung 100a zwischen der Datenbank 100 und dem zentralen Steuerkern 150 ist, dass 150 einen ersten Plan 10 aus der Datenbank entnimmt und auf der Darstellungs-Einrichtung 110 darstellt. Eine Rückspeicherung dieses Plans oder weiterer Pläne in die Datenbank 100 ist in der Regel nicht vorgesehen und die von dem - auch steuernden - zentralen Rechenkern 150 umgerechneten Pläne werden in dem temporären Speicher 120 gespeichert.

Ist aufgrund der Betriebsweise des Systems und der Benutzerführung ein kausaler Folgeplan 20 aus dem Eingangsplan (dem ersten Plan 10) entstanden, kann dieser auf Knopfdruck, beispielsweise mit der Schaltfläche 47 der Figur 12, in den Datenmanager 160 übertragen und dort zwischengespeichert werden. Diese Zwischenspeicherung wird durch nicht näher dargestellte technische Interfaces 161 in Einstellgrößen umgesetzt, welche dem technischen Gerät TG zugeführt werden. Mögliche technische Einstellparameter sind Zeitdauern, Intensitäten, Vorgaben für Multileaf-Kollimatoren oder Winkeleinstellungen für einen um den Patienten herum drehend veränderbaren Strahlungskopf. Die Arten der Einstellung des TG sind bekannt, die Geräte TG auch, so dass nur die Schnittstelle 161 an die bekannten Einstellmöglichkeiten anzupassen ist. Welche Einstellungen indes vorgenommen werde, ist Sache der Beispiele dieser Beschreibung.

Um das Ziel und Ergebnis dieser Arbeitsweise der Figur 1 oder des Systems der Figur 1, das in der beschriebenen Weise arbeitsfähig ist, vorweg zu stellen, soll auf Figur 12 verwiesen werden.

Figur 12 ist in der Anordnung der Einzelbilder 70', 72, 74, 76 sowie der Einstelleinrichtungen 30, 40 vergleichbar, nur wird ein anderer Plan 20 dargestellt, der aufgrund anderer Einstellungen der "Slider" 30, 40 zustande kommt. Dieser Plan 20 wird nach Betätigen der Schaltfläche 47 in den Datenmanager 160 übertragen und über das Interface 161 dem technischen Gerät TG zugeführt.

Figur 2 veranschaulicht das Planvolumen Z und die Außenhülle 10a der kleinen, lokalen Feinheit z, welche durch Anwahl des Initialvoxels z1 bestimmt wird. In dem schematischen Bild ist der Slice y derjenige mit der Nummer 57, so dass bei Slices y₁ bis yₙ der Slice y₅₇ das Initialvoxel enthält. Dargestellt ist diese Auswahl in Figur 6, wo im rechten oberen Teilbild 72 im Bereich der rechten Ohrspeicheldrüse 60 der Mauszeiger auf das Initialvoxel z1 zeigt. Nichts anderes repräsentiert Figur 2 schematisch, nur mit Blick von der Seite, nicht von oben.

Der Benutzer hat diese Stelle z1 aufgrund seiner Erfahrungen, Wünsche oder Ziele ausgewählt und möchte an dieser Stelle, die eine relativ hohe Dosis zeigt, lokal die Dosis herabsetzen, unter wesentlicher Beibehaltung des übrigen Plans, für den das DVH-Diagramm im linken oberen Teilbild 70 steht.

In gleicher Weise hätte der Benutzer das Initialvoxel z1 auch in einer der anderen Schnittdarstellungen der Figur 6 identifizieren können. Vom Ergebnis her wird bei Auswahl des Initialvoxels z1 in einer der drei Isodosis-Darstellungen 72, 74, 76 als Teilbilder der Figur 6 ein Folgebild der Figur 7 angezeigt. Dies mit der Darstellungseinrichtung 110, oder aber einer parallelen Darstellungseinrichtung 111.

Das aufscheinende Fenster 77 gibt nähere Auskunft zu dem "angeklickten" (durch Zeigen/Klicken mit einem Cursor identifizierten) Initialvoxel z1. Die zur Einstellung stehenden "Optionen" im Fenster 77 werden in Einstellfeldern vorgegeben. Es wird die Dosis des Voxels angegeben, der Ort dieses Voxels (rechte Ohrspeicheldrüse) und es können zwei Werte vorgegeben werden, die in Einstellfeldern 70a, 70b vom Benutzer eingegeben werden können. Der Benutzer kann die Größe einer Voxelgruppe definieren, die dem Volumen z entspricht. Im Beispiel ist das durch Eingabe einer halben Kantenlänge eines Würfels geschehen, der das geklickte Voxel enthält. Das wird im Feld 77b eingegeben. Der Benutzer kann eine gewünschte Dosis im Feld 77a eingeben, die für die gesamte Voxelgruppe z gelten soll. Im Beispiel ist als Wert 34 eingegeben. Ziel ist es also, die Dosis von 40gy auf 34gy herabzusetzen.

Eine Freigabe dieser Vorgabe erfolgt im Beispiel durch Betätigen der Schaltfläche 77c. Nach Freigabe, die einer gewollten Dosisänderung entspricht und ein Volumen definiert, innerhalb dessen diese Änderung erfolgen soll, erscheint das Folgefenster 78.

Es erscheint in Figur 8 das weitere Fenster 78, in dem eine Vielzahl anderer Parameter eingestellt werden können. Diese Parameter bestimmen die Umrechnung, die vom ersten Plan 10 ausgehend erfolgen soll. Die Umrechnung erfolgt mit der Verfahrensvorschrift von Philipp Süss, wie eingangs mit Fundstelle belegt. Es werden mathematische Gewichte eingestellt, die zunächst mit einer Voreinstellung vorgeschlagen werden. Die Voreinstellungen sind aus dem Bild ersichtlich und betreffen Kugelschalen und Gewichte. Die Gewichte steigen mit einem zunehmenden Abstand vom Initialvoxel und erhöhen den Lokalisierungsgrad bei der Umrechnung.

Das Feld 78 kann ein-, zwei oder mehrfach erscheinen.

Erscheint es ein zweites Mal kann mit anderen mathematischen Gewichten gerechnet werden, beispielsweise ohne solche Gewichte.

Es ist ebenso möglich, mit zwei deutlich unterschiedlichen Gewichten zwei Umrechnungen durchzuführen, die von dem ersten Plan 10 in einen ersten Navigationsplan 11 bzw. in einen zweiten Navigationsplan 12 münden. Den in der Regel größten Unterschied dieser beiden Navigationspläne 11,12 erhält der Benutzer, wenn der erste Navigationsplan ohne mathematische Gewichte berechnet wird und der zweite Navigationsplan mit den eingestellten mathematischen Gewichten berechnet wird.

Das Bestätigen auf dem Betätigungsfeld 78c startet die Umrechnung.

Ziel der Umrechnung ist die Veränderung der Dosis in dem lokal begrenzten Volumen z mit der Hülle 10a. In diesem kleinen Volumen befindet sich das Initialvolumen. Im Beispiel ist von 40gy auf 23gy zu reduzieren.

In einer Variante der Berechnung kann die kleine Voxelgruppe z vom System darauf untersucht werden, ob sie bei Beseitigung eines critical Spot im Zielbereich Voxel aus dem Risikobereich, oder bei Beseitigung eines critical Spot im Risikobereich Voxel aus dem Zielbereich enthält. Die Anzahl der Voxel in der Voxelgruppe ist maximal 500, bevorzugt weniger als 350, oder mit Prozentangaben bemessen nicht mehr als 5% der angesetzten Voxel des Planvolumens. Das System kann die Anzahl der umzurechnenden Voxel um diese dem jeweils anderen Bereich zugehörigen Voxel reduzieren, so dass die Anzahl der Voxel in der Voxelgruppe sinkt. Als Begründung kann dazu angegeben werden, dass für die Beseitigung eines Critical Spots in einem Bereich, d.h. einem Risikobereich oder Zielbereich, die Voxel des jeweils anderen Bereichs nicht von Relevanz sind.

Als Folge der Umrechnung gemäß den Figuren 7 und 8 steht in der Figur 9 funktionsmäßig zumindest ein Slider 40 als Beispiel einer Bedienhilfe zur Verfügung. Dieser bildet die "erste Bedienhilfe". Ein Stellknopf X40 kann zwischen dem linken Rand 41 und dem rechten Rand 42 des Sliders bewegt werden. Im Beispiel der Figur 9 steht X40 am rechten Rand. Stellknopf X40 wählt dabei einen Zwischenplan aus, der dieser (Zwischen-) Stellung im Slider 40 zugewiesen ist.

In einem Beispiel kann dieses der zweite Navigationsplan 12 sein. Es kann aber ebenso der erste Navigationsplan 11 sein. Dieses Beispiel ist in Figur 10 dargestellt.

In beiden Figuren ist zusätzlich eine Variante eingebaut, die über die Verwendung einer zweiten Bedienhilfe 30 möglich wird. Diese Bedienhilfe 30 legt auf ihre beiden Endstellungen den ersten Navigationsplan 11 bzw. den zweiten Navigationsplan 12. Dort, wo der zweite Einstellknopf X30 steht, wird ausgewählt, welcher Zwischenplan der rechten Endlage 42 der ersten Einstellhilfe 40 zugewiesen wird.

Mit Figur 8, eigentlich der zweimaligen Anwendung der Umrechnung gemäß Figur 8 mit unterschiedlichen mathematischen Gewichten, wurden zwei Navigationspläne errechnet, die 11 und 12 heißen.

Geht man davon aus, dass der erste Navigationsplan ohne Gewichte errechnet wurde, liegt er an der rechten Endstellung 31 der zweiten Bedienhilfe 30, die im Beispiel auch als Slider ausgeführt ist. An der linken Endstellung 32 liegt der zweite Navigationsplan 12, der mit mathematischen Gewichten berechnet wurde. Aufgrund der Stellung des Knopfes X30 am linken Rand des zweiten Sliders 30 wird der zweite Navigationsplan mit den mathematischen Gewichten ausgewählt und der rechten Endstellung 42 des ersten Sliders 40 zugewiesen. Steht hier der Einstellknopf X40, wird auf dem Planabschnitt P der zweite Navigationsplan 12 dargestellt, und zwar im Beispiel repräsentiert durch die vier Teilbilder, die zuvor angesprochen waren.

Ersichtlich ist, dass die Teilbilder sich mit unterschiedlichen Grauwerten in den Intensitäten von den Ausgangsbildern der Figuren 4a, 4b und 4c unterscheiden. Aufgrund der Verwendung der mathematischen Gewichte beim zweiten Navigationsplan sind die Planänderungen lokaler ausgefallen. Das kann im Vergleich zu Figur 10 ersehen werden.

In Figur 10 ist mit den beiden Bedienhilfen eine solche Einstellung gewählt, bei der der rechten Endstellung der Bedienhilfe 40 der erste Navigationsplan zugewiesen ist, weil der Einstellknopf X30 der ersten Bedienhilfe 30 am rechten Anschlag 31 steht, der repräsentativ für den ersten, nicht mit mathematischen Gewichten berechneten Navigationsplan 11 ist. Konsequenterweise wird im Planabschnitt P der erste Navigationsplan 11 dargestellt.

Ein Wort zu den DVH-Diagrammen 70' der Figuren 9 und 10. Es ist jeweils ein Paar an Kurvenverläufen für ein Objekt oder einen Bereich im Planvolumen dargestellt, den ersten und zweiten Tumor, die linke und rechte Schilddrüse und einige andere Organe, zu denen es DVH-Kennlinien gibt, die das System der Figur 1 errechnet.

Ein jeweiliges Kurvenpaar zeigt den Unterschied zwischen dem ursprünglichen Eingangsplan 10 der Figur 5 und dem über die Slider 30, 40 neu gefundenen kausalen Folgeplan, der aber nur - ohne Mischungen - dem ersten Navigationsplan 11 **oder** dem zweiten Navigationsplan 12 entsprechen kann.

Ein Benutzer kann an einer Bewertung des Teilbilds 70' der beiden Figuren 10 und 11 erkennen, dass die Dosis im Zielvolumen, dem Tumor (die rechten Kurven 80, 81 im DVH-Diagramm 70 der Figur 3 und 70' der Figuren 10/11) zurückgegangen ist, und zwar bei der Verwendung der mathematischen Gewichte, also dem zweiten Navigationsplan.

Ohne diese mathematischen Gewichte und demzufolge dem ersten Navigationsplan 11 ergibt sich eine deutliche Erhöhung der Dosis für das Zielvolumen als Tumor. Das eröffnet für den Benutzer eine weitere Einstellmöglichkeit, diejenige der Navigation mit dem zweiten Slider 30 zwischen dem ersten und dem zweiten Navigationsplan 11/12 im Wege der Interpolation.

Das veranschaulicht Figur 11. Figur 11 zeigt eine Position des Einstellknopfes X30 zwischen den beiden Endstellungen 31, 32. Diese Zwischenposition ist 33 benannt. Hier ergibt sich ein dritter Navigationsplan 13, der gemäß den Ausführungen zu Figuren 9 und 10 der rechten Endstellung 42 der ersten Bedienhilfe 40 zugewiesen wird. Hier steht auch der Einstellknopf X40, so dass seine Einstellung den dritten Navigationsplan der Einstellposition 33 auf den Planabschnitt P bringt. Entstanden ist der dritte Navigationsplan 13 durch Interpolation zwischen den Navigationsplänen 11 und 12 entsprechend der Stellung des Einstellknopfs X40 und seinem Abstand von der linken und rechten Endstellung.

Das DVH-Diagramm 70' im linken oberen Teilbild zeigt eine deutliche Verbesserung. Der Nutzer erhält einen kausalen Folgeplan, den er durch Veränderung des Einstellknopfs jeder der beiden Bedienhilfen 30, 40 oder 130, 140 in einem Nahezu-Kontinuum entwickeln kann. Es sind dazu nicht nur die beiden Endstellungen jeder der Bedienhilfen, sondern auch eine oder mehrere Zwischenstellungen in jeder Bedienhilfe vorgesehen, von denen eine Zwischenstellung 33 mit Figur 11 erläutert wurde. Weitere solche Zwischenstellungen bilden weitere Interpolationen zwischen den jeweiligen Endwerten einer der Bedienhilfen, und zwar sowohl 30, wie auch 40.

In der linken Bedienhilfe 40 liegt am linken Ende der erste Plan 10. Figur 12 zeigt, dass auch hier eine Interpolation zwischen diesem ersten Plan 10 und demjenigen (Navigations-) Plan möglich ist, der am Endanschlag 42 gelegen ist. Diesen gibt die rechte Bedienhilfe 30 vor, durch Einstellen auf einem Zwischenwert.

Der rechte Slider ermöglicht die Interpolation zwischen dem ersten und dem zweiten Navigationsplan, sorgt für einen interpolierten Plan an der Stelle 33 (durch die Lage des Einstellknopfs X30 als dritter Navigationsplan 13) und der linke Slider 40 interpoliert zwischen dem ersten Plan 10 und dem "dritten Navigationsplan" genannten Mischplan, der hier der Sliderstelle 33 entspricht.

Beide Slider veranlassen nur eine Interpolation, die vom Rechenkern 150 vorgenommen wird, und zwar während der Betätigung der Einstellknöpfe der Slider entlang ihrer mehreren Zwischenpositionen. Diese Zwischenpläne, die aufgrund der Bewegung der beiden Slider 30, 40 entstehen, werden in den Zwischenspeicher 120 abgelegt und können bei entsprechender Position eines Sliders wieder aufgerufen werden.

Hat der Benutzer einen für ihn günstigen Plan gefunden, den er an dem DVH-Diagramm 70' als "gut" identifiziert und auch nach den übrigen Isodosis-Teilbildern als sinnvoll erachtet, kann er diesen günstigen Plan in den Datenmanager 160 exportieren, durch Betätigen der Schaltfläche 47. Dieser Plan, der im Beispiel der Figur 12 der kausale Folgeplan 20 ist, ist das gewünschte Ergebnis.

Es entsteht aus mehreren - slider-veranlassten - Interpolationen und aus der zuvor erfolgten Umrechnung des ersten Plans in zwei Navigationspläne 11, 12 mit unterschiedlichen mathematischen Gewichten, aber alles gestützt auf den einen Eingangsplan 10.

Es können zusätzliche Einstellhilfen oder Anzeigen Verwendung finden, die mit 45 und 46 benannt sind. Die Anzeige 46 stellt ein Maß für die Größe des Bereichs an, der im Vergleich zum alten Plan verändert wurde.

Ein Beispiel ist das arithmetische Mittel über den Abstand aller Voxel zum Initialvoxel z1, deren Änderungen mehr als 0,1 gy beträgt. Im Beispiel der Figur 13 wird als Maß für die Bereichsgröße der Abweichungswert 41 von der Anzeige 46 dargestellt. In den beiden Bildern der Figuren 10 und 11 wäre dieser Abweichungswert 60. Je kleiner dieses Maß oder diese Größenangabe ist, desto geringer ist die Abweichung von dem ersten Plan 10 (Eingangsplan), aber unter Berücksichtigung der Änderungsvorgabe in der kleinen/lokalen Gruppe von Voxeln z, die mit Figur 7 eingestellt wurde.

Der entstandene Folgeplan der Figur 13 ist also günstiger, was die Nähe zum ersten Plan 10 angeht. Wenn der Benutzer diesen Folgeplan befürwortet, wird er als kausaler Folgeplan 21 über die Schaltfläche 47 in den Datenmanager 160 übertragen und dort für die Weitergabe an das einzustellende Gerät TG in dem Interface 161 umgesetzt.

Besonders deutlich an Figur 13 ist die Nähe der paarweise dargestellten DVH-Kennlinien, z.B. der Kennlinien 80, 80'für das Zielvolumen des ersten Tumors.

Diese ist in Figur 14 nochmals herausvergrößert.

Es ist in Figur 14 zwischen den beiden Kennlinien 80, 80' kaum ein Unterschied zu sehen und auch der zweite Tumor mit den beiden Kennlinien 81, 81' lässt kaum einen Unterschied im Verlauf der DVH-Kennlinienschaar erkennen. Leichte Unterschiede sind bei den Kurven (auch: Kennlinien) zweier anderer Organe zu erkennen, die das Rückenmark mit den DVH-Kennlinien 82 und 82' und die rechte Ohrspeicheldrüse mit den DVH Kennlinien 83, 83' repräsentieren.

Nahe oder in der rechten Ohrspeicheldrüse lag das Initialvoxel z1. Die Veränderung ihrer DVH Kennlinie 83 nach Figur 5 zu der Einstellung nach Figur 13 ist deutlicher zu sehen und mit 83' benannt.

Möchte man ein Maß dafür angeben, wie die Kennlinien der DVH-Verteilung sich in ihrer Nähe zu dem ursprünglichen ersten Plan 10 verhalten, könnte man in einer ersten Näherung sagen, dass sie, die Kurven (Kennlinien) des DVH-Teilbilds 70, nicht mehr als 5% abweichen sollen. Bei dem Teilbild 70" ist das sehr gut gegeben, was auch optisch nachvollzogen werden kann und in der Figur 14 als Ausschnittsvergrößerung deutlich wird. Dies betrifft natürlich nur den kausalen Folgeplan, der aus den Stufen oder Schritten, die im Rahmen der Ausführungsbeispiele erläutert wurden, entsteht. Andere Pläne, auch der erste oder zweite Navigationsplan können deutlicher abweichen, da sie selbst nicht den kausalen Folgeplan 20 oder 21 darstellen müssen, sondern nur eine Hilfe zur Navigation quasi in einem Kontinuum eröffnen, bei dem drei Pläne als "Eckpläne" zur Verfügung stehen, der erste Plan 10 und zwei daraus umgerechnete Navigationspläne 11, 12.

## Patentansprüche

1. **Verfahren** zum Anpassen einer Dosisverteilungs-Einstellung eines technischen Geräts der Tumortherapie,
- Auslesen eines ersten Plans (10) aus einem Datenspeicher (100);
- Darstellen des ersten Plans (10) einer Dosisverteilung in einem Planvolumen (Z) auf einer Darstellungseinrichtung (110) für eine mögliche Einstellung des technischen Geräts der Tumortherapie zur Abgabe der eingestellten Dosisverteilung an einen Patienten;
wobei eine erste Bedienhilfe (40) und eine zweite Bedienhilfe (30) je zwei Endstellungen (41,42) aufweisen, und die erste Endstellung der ersten Bedienhilfe (40) dem ersten Plan (10) entspricht, wobei die zweite Bedienhilfe (30) einen Navigationsplan (11,12,13) der zweiten Endstellung (42) der ersten Bedienhilfe (40) zuordnet;
- zum Zwecke der Änderung des ersten Plans (10) in zumindest einer volumenmäßig spezifischen Feinheit (10a) und Erstellung oder Ermittlung eines kausalen Folgeplans zur Einstellung am/des technischen Gerät(s) der Tumortherapie;
- Vorgabe eines neuen Dosiswerts für eine lokale Gruppe von Voxeln (z) in der oder als die volumenmäßig spezifizierte Feinheit (10a) an einer bestimmten Stelle des Planvolumens (Z), welche diesen neuen Dosiswert nicht aufweist;
- wobei die bestimmte Stelle durch Auswahl eines Initialvoxels (z1) festgelegt wird, das in einer Schicht (yi) des Planvolumens gelegen ist;
- Umrechnen des ersten Plans (10) in einen ersten Navigationsplan (11), welche Umrechnung den ersten Plan (10) und die Dosisänderung in der lokalen Voxelgruppe beachtet;
- wobei der erste Plan (10) in einen zweiten Navigationsplan (12) umgerechnet wird, entsprechend der ersten Umrechnung und mit Vorgabe von mathematischen Gewichten bei der Umrechnung, so dass erster und zweiter Navigationsplan (11,12) unterschiedlich sind, wobei die mathematischen Gewichte mit einem zunehmenden Abstand von dem Initialvoxel (z1) steigen und der zweite Navigationsplan (12) lokal begrenztere Änderungen gegenüber dem ersten Navigationsplan (11) aufweist;
- und die zweite Bedieneinrichtung (30) mehrere Zwischenstellungen besitzt, deren jede einem Zwischenplan entspricht, wobei jeder der Zwischenpläne einer Interpolation zwischen dem ersten Navigationsplan (11) und dem zweiten Navigationsplan (12) entspricht, und eine ausgewählte Zwischenstellung (33) einen jeweiligen dritten Navigationsplan (13) als Mischplan festlegt;
- wobei zwischen den beiden Endstellungen der ersten Bedienhilfe (40) mehrere Zwischenstellungen bestehen, deren jede einem Zwischenplan entspricht, und jeder der Zwischenpläne einer Interpolation aus dem ersten Plan (10) in Richtung des Navigationsplans (11,12,13) entspricht;
- Darstellen eines Plans (10,11,12,13) auf der Darstellungseinrichtung (110), abhängig von einer Einstellung der ersten Bedienhilfe (40).

2. Verfahren nach Anspruch 1, wobei eine Mitteneinstellung der ersten Bedienhilfe (40) einen Zwischenplan auf der Darstellungseinrichtung (110) anzeigt, der interpoliert zwischen dem ersten Plan (10) und dem dritten Navigationsplan (13) liegt.

3. Verfahren nach einem der Ansprüche 1 oder 2, wobei jeder der Zwischenpläne einer Dosisverteilung in dem Planvolumen (Z) für eine entsprechende Einstellung des technischen Geräts entspricht.

4. Verfahren nach Anspruch 1, wobei die interpolierten Zwischenpläne nicht in der Datenbank (100) gespeichert werden.

5. Verfahren nach Anspruch 1, wobei die lokale Gruppe von Voxeln (z) weniger als 500 Voxel umfasst und das Planvolumen mehr als 2000 Voxel umfasst;

6. Verfahren nach Anspruch 1 oder 5, wobei die lokale Gruppe weniger als 5% des Planvolumens (Z) einnimmt.

7. Verfahren nach Anspruch 1, wobei die mathematischen Gewichte so gestaltet werden, dass sie über verschiedenen Richtungen vom Initialvoxel (z1) ausgehend in Abhängigkeit vom jeweiligen Gewebe in dieser Richtung gesetzt oder vorgegeben werden.

## Claims

1. **Method** for adjusting a dose distribution setting of a technical device for tumor therapy,
- reading a first plan (10) from a data memory (100);
- displaying the first plan (10) of a dose distribution in a plan volume (Z) on a display device (110) for a possible setting of the technical apparatus of tumor therapy for delivering the set dose distribution to a patient;
wherein a first operating aid (40) and a second operating aid (30) each have two end positions (41, 42), and the first end position of the first operating aid (40) corresponds to the first plan (10), wherein the second operating aid (30) assigns a navigation plan (11, 12, 13) to the second end position (42) of the first operating aid (40);
- for purpose of changing or amending the first plan (10) in at least one volume-specified fineness (10a) and drawing up or determining a causal follow-up plan for adjustment on/at the technical device of tumor therapy;
- presetting of a new dose value for a local group of voxels (z) in or as the volume-specified fineness (10a) at a certain position of the planned volume (Z), which does not have this new dose value,
- wherein the specific location is determined by selecting an initial voxel (z1), which is located in a layer (yi) of the plan volume;
- conversion of the first plan (10) into a first navigation plan (11), which conversion takes into account the first plan (10) and the dose change in the local voxel group;
- wherein the first plan (10) is converted into a second navigation plan (12) in accordance with the first conversion and with presetting of mathematical weights in the conversion, so that first and second navigation plans (11, 12) are different, wherein the mathematical weights increase with increasing distance from the initial voxel (z1) and the second navigation plan (12) has more locally limited changes compared with the first navigation plan (11);
- and the second operating device or aid (30) has a plurality of intermediate positions, each of which corresponds to an intermediate plan, each of the intermediate plans corresponding to an interpolation between the first navigation plan (11) and the second navigation plan (12), and a selected intermediate position (33) defining a respective third navigation plan (13) as a mixed plan;
- wherein between the two end positions of the first operating aid (40) there are a plurality of intermediate positions, each of which corresponds to an intermediate plan, and each of the intermediate plans corresponds to an interpolation from the first plan (10) in the direction of the navigation plan (11, 12, 13);
- displaying a plan (10, 11, 12, 13) on the display device (110), depending on a setting of the first operating aid (40).

2. Method according to claim 1, wherein a center setting of the first operating aid (40) displays an intermediate plan on the display device (110) that lies interpolated between the first plan (10) and the third navigation plan (13).

3. Method according to one of claims 1 or 2, where each of the intermediate plans corresponds to a dose distribution in the plan volume (Z) for a corresponding adjustment of the technical device.

4. Method according to claim 1, whereby the interpolated intermediate plans are not stored in the database (100).

5. Method according to claim 1, wherein the local group of voxels (z) comprises less than 500 voxels and the plan volume comprises more than 2000 voxels;

6. Method according to claim 1 or 5, where the local group takes up less than 5% of the plan volume (Z)

7. Method according to claim 1, wherein the mathematical weights are designed in such a way that they are set or predetermined in this direction over different directions starting from the initial apex (z1) depending on the respective tissue.

## Revendications

1. **Procédé** pour ajuster un réglage de distribution de dose d'un dispositif technique pour la thérapie des tumeurs,
- lecture d'un premier plan (10) à partir d'une mémoire de données (100) ;
- affichage du premier plan (10) d'une distribution de dose dans un volume de plan (Z) sur un dispositif d'affichage (110) pour un réglage éventuel de l'appareil technique de thérapie tumorale pour délivrer la distribution de dose réglée à un patient ;
dans lequel une première aide de fonctionnement (40) et une deuxième aide de fonctionnement (30) présentent chacune deux positions finales (41, 42), et la première position finale de la première aide de fonctionnement (40) correspond au premier plan (10), dans lequel la deuxième aide de fonctionnement (30) associe un plan de navigation (11, 12, 13) à la deuxième position finale (42) de la première aide de fonctionnement (40);
- pour la modification du premier plan (10) dans au moins une finesse spécifique au volume (10a) et pour l'élaboration ou la détermination d'un plan de suivi causal pour l'adaptation sur le ou les dispositifs techniques de la thérapie tumorale;
- préréglage d'une nouvelle valeur de dose pour un groupe local de voxels (z) dans ou comme finesse (10a) spécifiée par le volume à une certaine position du volume prévu (Z), qui ne présente pas cette nouvelle valeur de dose
- dans lequel l'emplacement spécifique est déterminé en sélectionnant un initial voxel (z1), qui est situé dans une couche (yi) du volume du plan ;
- conversion du premier plan (10) en un premier plan de navigation (11), laquelle conversion tient compte du premier plan (10) et du changement de dose dans le groupe local de voxels;
- dans lequel le premier plan (10) est converti en un deuxième plan de navigation (12) conformément à la première conversion et à un préréglage de poids mathématiques dans la conversion, de sorte que les premier et deuxième plans de navigation (11, 12) sont différents, dans lequel les poids mathématiques augmentent avec une distance croissante par rapport au voxel initial (z1) et le deuxième plan de navigation (12) présente des modifications plus limitées localement par rapport au premier plan de navigation (11);
- et le deuxième dispositif de commande (30) présente une pluralité de positions intermédiaires, dont chacune correspond à un plan intermédiaire, chacun des plans intermédiaires correspondant à une interpolation entre le premier plan de navigation (11) et le deuxième plan de navigation (12), et une position intermédiaire sélectionnée (33) définissant un troisième plan de navigation respectif (13) comme plan mixte;
- dans lequel, entre les deux positions d'extrémité de la première aide à la navigation (40), il existe plusieurs positions intermédiaires, dont chacune correspond à un plan intermédiaire, et chacun des plans intermédiaires correspond à une interpolation du premier plan (10) en direction du plan de navigation (11, 12, 13);
- affichage d'un plan (10, 11, 12, 13) sur le dispositif d'affichage (110), en fonction d'un réglage de la première aide à l'utilisation (40).

2. Procédé selon la revendication 1, dans lequel un réglage central de la première aide à la navigation (40) affiche sur le dispositif d'affichage (110) un plan intermédiaire qui est interpolé entre le premier plan (10) et le troisième plan de navigation (13).

3. Procédé selon l'une des revendications 1 ou 2, où chacun des plans intermédiaires correspond à une distribution de dose dans le volume du plan (Z) pour un ajustement correspondant du dispositif technique.

4. Procédé selon la revendication 1, les plans intermédiaires interpolés n'étant pas stockés dans la base de données (100).

5. Procédé selon la revendication 1, dans lequel le groupe local de voxels (z) comprend moins de 500 voxels et le volume du plan comprend plus de 2000 voxels.

6. Procédé selon la revendication 1 ou 5, lorsque le groupe local occupe moins de 5 % du volume du régime (Z).

7. Procédé selon la revendication 1, dans lequel les poids mathématiques sont conçus de telle manière qu'ils sont réglés ou prédéterminés dans cette direction sur différentes directions à partir de l'apex initial (z1) en fonction du tissu respectif.
